# FASCICULE DE BREVET EUROPEEN

(11) **EP 0 494 297 B1**
(45) Date de publication et mention de la délivrance du brevet: **18.10.1995**
(21) Numéro de dépôt: 91914274.5
(22) Date de dépôt: 25.07.1991
(51) Int. Cl.: A61K 9/18, A61K 9/20, A61K 31/60

(54) **PROCEDE DE PREPARATION GALENIQUE D'UNE COMPOSITION THERAPEUTIQUE NOTAMMENT A BASE D'ASPIRINE**
VERFAHREN ZUR GALENISCHEN HERSTELLUNG EINES THERAPEUTISCHEN MITTELS, INSBESONDERE AUF ASPIRINBASIS
GALENICAL PREPARATION METHOD FOR PREPARING A THERAPEUTICAL COMPOSITION PARTICULARLY BASED ON ASPIRIN

(30) Priorité: 26.07.1990 EP 90402150
(43) Date de publication de la demande: 15.07.1992
(73) Titulaire: Perovitch, Philippe, 33700 Mérignac (FR); Maury, Marc, F-33000 Bordeaux (FR)
(72) Inventeur: Perovitch, Philippe, 33700 Mérignac (FR); Maury, Marc, F-33000 Bordeaux (FR)
(74) Mandataire: Wagret, Jean-Michel
(86) Numéro de dépôt international: FR9100619
(87) Numéro de publication internationale: WO9201444

(56) Documents cités:
- EP-A- 0 287 488
- BE-A- 690 635
- FR-A- 2 649 611
- GB-A- 1 349 158
- GB-A- 1 468 557
- GB-A- 2 188 843
- US-A- 4 206 209

## Description

L'invention concerne la présentation galénique d'une composition thérapeutique.

On connait par le brevet GB 1 349 158 la préparation de compositions pharmaceutiques sous forme de poudre, dont chaque grain est formé d'une particule enrobée d'un film du produit actif par immersion des particules dans une émulsion contenant le produit actif, suivie d'un séchage par atomisation.

On a cherché à obtenir des produits actifs à l'état microdispersé et répartis à l'intérieur d'un substrat lui-même de structure réceptrice en mettant en solution le principe actif (insoluble ou très peu soluble dans l'eau) dans un solvant organique; le substrat ainsi imprégné de la solution est ensuite soumis à une phase d'évaporation du solvant, le produit actif étant ainsi contraint à recristalliser à l'intérieur de la structure support sous forme de cristaux de faibles dimensions et présentant par conséquent la surface spécifique importante recherchée.

Un procédé de ce type est notamment décrit dans la demande de brevet européen 0 287 488, mais malgré l'intérêt théorique de ce procédé, il est apparu que sa mise en oeuvre rencontrait des difficultés pratiques au niveau de l'exploitation de la fabrication, dans un cadre industriel.

La principale difficulté résulte du manque d'homogénéité entre le substrat et les particules constitutives des microcristaux adsorbés au sein et à la surface du substrat notamment à la surface des anfractuosités de ce dernier.

A défaut d'une liaison homogène entre les deux solides, leur cohésion ne peut être assurée lors des phases que doivent successivement subir le substrat et son contenu pour être acheminés jusqu'à la forme galénique définitive acceptable(comprimé ou tablette).

On constate en effet que lors de la phase d'évaporation sous vide, par exemple, la force d'aspiration nécessaire pour assurer un traitement rentable et une évacuation rapide du solvant, aboutit à un démélange de l'ensemble, les microcristaux constitués, comme recherchés, d'une poussière infinitésimale, de l'ordre de quelques microns, sont entraînés ou élués par le courant gazeux qui les aspire et qui appauvrit par conséquent le substrat en entraînant une perte notable en produits actifs.

Ce phénomène se retrouve lors des phases ultérieures de traitement. La différence de taille entre le substrat et les microcristaux entraîne des problèmes difficiles lors de la mise en comprimés. La présence des microcristaux constitue un surcroit de forces de frottement et d'adhérence et limite en effet les propriétés d'écoulement du mélange dans les presses et la mise en oeuvre de machines de production à cadences élevées et à hauts rendements est plus que compromise.

Enfin, la surface spécifique élevée des microcristaux, qui est souhaitable dans le cadre du but recherché par l'invention, augmente les phénomènes de grippage rencontrés lors de la fabrication des comprimés, les lubrifiants utilisés classiquement pour la fabrication des comprimés n'étant pas suffisamment efficaces pour assurer le maintien des conditions de cadences et de qualité souhaitables, en particulier lorsque les phénomènes de grippage devenant trop importants, provoquent des défauts de qualité des comprimés. De sorte que la mise en oeuvre d'une technique de microdispersion à forte concentration sur un substrat n'a pu être pratiquement obtenue industriellement malgré l'intérêt qu'elle représenterait sur le plan galénique et commercial.

L'invention permet de remédier à cet inconvénient et elle prévoit des conditions qui permettent d'assurer l'homogénéité du substrat et des microcristaux adsorbés sur le support, permettant le traitement de ce dernier par des machines usuelles de fabrication évitant ainsi les inconvénients qui ont été décrits précédemment.

A cet effet, l'invention vise une forme de mise en oeuvre du procédé de réalisation du microdispersion qui assure en premier lieu une homogénéité dans la répartition des microcristaux au sein d'un substrat devenu poreux et spongieux au cours des opérations, en partant d'un état réticulé, et garantit la stabilité de ces microcristaux une fois mis en place, en prévenant toute perte ou démélange en cours de fabrication.

L'invention concerne plus particulièrement un procédé de préparation d'une composition thérapeutique tel que défini dans la revendication 1 qui suit.

On obtient grâce à la mise en oeuvre de l'invention, lors de la formation des microcristaux, d'une part une limitation de leur envergure cristalline, et d'autre part le maintien en place et la solidarisation ferme de ces microcristaux sur la paroi du substrat grâce à la présence dudit adjuvant lequel assure une cimentation des microcristaux sur leur paroi d'adsorption en évitant tout entraînement parasite et toute dispersion ultérieure lors des phases subséquentes de fabrication; mais encore, on obtient une régularisation de la forme externe, générale, granulée du substrat, ce qui facilite son utilisation industrielle.

Plus particulièrement, le procédé de l'invention est caractérisé en ce que le substrat, après absorption de la solution, est soumis à une opération d'évacuation du solvant à température ambiante par réduction de la pression atmosphérique, c'est-à-dire par séchage sous vide; dès lors le substrat acquiert par cette application une structure poreuse spongieuse et régulière qui recèle les microcristaux de principe actif.

Selon une particularité, la phase de conditionnement comporte l'homogénéisation du substrat suivie d'une compression en vue de la formation de doses pour prises unitaires et constituant des pastilles, comprimés, tablettes, granulés ou dragées.

Et l'on comprend que lors de la fabrication ainsi mise en oeuvre, l'adjuvant grâce à ses propriétés particulières atténue les forces de frottement issues des surfaces en contact et permettra d'apporter au sein de la masse même du substrat, comme sur les parois externes de la tablette ou du comprimé en formation, les propriétés de glissement des éléments et parois les unes sur les autres propres à assurer la texturation finale.

L'invention est plus particulièrement applicable à la présentation d'une spécialité antalgique ou anti-inflammatoire à base d'aspirine à usage local ou à assimilation directe par les voies des muqueuses sublinguales, pour des spécialités à visée thérapeutique générale avantageusement appréhendées par la voie sublinguale.

Selon une autre caractéristique, le solvant binaire utilisé comporte à titre de solvant principal un alcool à faible tension de vapeur et plus spécialement un alcool monofonctionnel inférieur choisi dans la famille comportant le méthanol, l'éthanol, le butanol et le propanol.

Plus spécialement, on utilise un alcool possédant au départ une concentration de préférence supérieure à 90 °.

Plus spécialement encore, l'adjuvant intégré dans le solvant binaire et mis en solution dans ce dernier est constitué d'un polymère organique choisi pour ne pas limiter la solubilité du produit actif dans le solvant principal.

Plus spécialement, l'adjuvant est constitué d'un polymère organique, soluble dans les alcools inférieurs et dans l'eau.

Enfin, l'adjuvant est plus spécialement encore choisi dans la famille des polymères comportant :
- les Monoglycérides
- les Diglycérides
- les Polyéthylènes Glycols (PEG)
- Esters et Ethers des alcoolamines
- Esters et Ethers des Diethanolamines
- Esters et Ethers des Isopropanolamines
- Esters et Ethers des Monoéthanolamines
- Ethoxylamine

Dérivés éthoxylés des acides gras
Polyéther-alcool d'alkylamyl
Polyéther-alcool d'alkanolamide
Les Esters et Ethers et notamment :
Esters acides de mono et diglycérides
Esters d'acides gras
Esters du Polyéthylène Glycol (PEG), notamment monolaurate, stéarate, distéarate de PEG.
Esters et Ethers du Propylène Glycol
Esters et Ethers du Polyglycérol
Esters et Ethers du Polyglycol
Esters et Ethers du Glycol
Dérivés Ethoxylés des alcools et alcools gras et Phénols.
Dérivés Propoxylés des alcools gras et alkylphénols.
Ethoxyalkylphénol
Esters du Sorbitane notamment Monostéarate de Sorbitane
Esters du Saccharose notamment Palmitate et Stéarate de Saccharose.
Polyoxyéthers de Glycol, de Sorbitane, de Glycérol
Lécithines et dérivés
Alkyloamide
Huile de Castor éthoxylée hydrogénée
Esters du Sorbitane éthoxylé
Esters de l'éthylène glycol
Copolymères et polymères de l'oxyde d'Ethylène et de Propylène
Dérivés de l'éthanolamide d'acides gras
Dérivés de diéthanolamide d'acides gras.
Polycondensats d'éthylène oxyde
Polycondensats de polypropylène glycol
Selon une autre caractèristique, l'adjuvant est introduit dans le système solvant binaire dans une proportion comprise entre 10 % et 60 % du poids total du solvant binaire.

Selon encore une autre particularité, le substrat sera choisi dans la famille comportant les hydrates de carbone alimentaires.

Et plus spécialement encore, le substrat est constitué par du sorbitol ou du mannitol.

Selon une forme de réalisation du procédé de l'invention, l'imprégnation et l'absorption de la solution par le substrat est poursuivie jusqu'à saturation et obtention d'une préparation homogène de la solution adsorbée dans le substrat, ceci tant au sein de chaque granule qu'au niveau des granules entre eux.

Et selon une caractéristique de mise en oeuvre du procédé, le substrat subit plusieurs cycles successifs d'enrichissement en microcristaux, chaque cycle comportant une phase d'imprégnation suivie d'une phase d'évaporation, les cycles étant répétés jusqu'à ce que la saturation en microcristaux soit optimale.

L'invention concerne également une spécialité médicamenteuse sous présentation galénique et constituée notamment d'une pastille, granule, dragée, comprimé ou analogue, apte à être sucé par l'utilisateur et à administration sublinguale, caractérisé en ce qu'il comporte au moins un produit actif sous forme microdispersée au sein d'un substrat, cette microdispersion ayant été obtenue conformément au procédé ci-dessus spécifié.

Et plus particulièrement la spécialité comporte un produit actif microdispersé au sein d'un substrat et inclus dans la structure spongieuse ou alvéolaire de ce dernier sous forme de microcristaux, la taille des microcristaux étant généralement inférieure à 10 microns.

Selon un exemple de réalisation de la spécialité selon les caractéristiques ci-dessus, le produit actif est constitué par de l'aspirine reconstituée sous forme de micro-cristaux au sein d'un support constitué de sorbitol.

Et plus particulièrement, la spécialité médicamenteuse ci-dessus décrite et à base d'aspirine microdispersée comporte un agent acidifiant tel que l'acide citrique, ou acidifiant équivalent.

De préférence, la composition de chaque tablette ou comprimé est basée sur un pourcentage d'aspirine sous forme de microcristaux compris entre 10 et 200 milligrammes sur un substrat tel que du sorbitol de poids compris entre 100 et 2 500 milligrammes.

Et selon une forme de réalisation plus particulière, la spécialité médicamenteuse est caractérisée en ce qu'elle comporte en combinaison synergique outre de l'aspirine sous forme microdispersée, un ou plusieurs produits actifs auxiliaires choisis dans les familles comportant :
- des antiseptiques (tels les ammoniums quaternaires, la chlorexhidine)
- des antibiotiques (tels que Bacitracine, Tyrothricine, Fusafungine)
- des antifongiques locaux (tels que miconazole, nystatine, amphotéricine B)
- des antiviraux (tel l'Acyclovir, l'Azido-thymidine (AZT), les interférons)
- des cicatrisants (allantoïne, azulène)
- des enzymes (tels lysozyme, papaïne, bromeline)
- des analgésiques (tels la procaïne, la tétracaïne, la stovaïne).

On a décrit ci-après quelques exemples de mises en oeuvre de l'invention appliquée notamment à la fabrication d'une spécialité thérapeutique à base d'aspirine, mais ledit procédé s'adapte de la même manière à un grand nombre de substances thérapeutiques ayant des caractéristiques physicochimiques comparables (faible solubilité dans l'eau, agressivité envers les tissus, mauvais goût, mauvaise tolérance) ou encore, une biodisponibilité imparfaite dans le tractus digestif ou encore une demi-vie biologique courte de quelques heures à quelques minutes, ceci à titre indicatif et non limitatif, comme le diltiazem, la nifédipine, la molsidomine, l'amiodarone, le bepridil, la prostacycline et ses analogues, la ticlopidine et ses analogues, ou toutes substances peptidiques à action anticancéreuse ou hormonale.

Selon une première forme de réalisation de l'invention, cette dernière comporte les différentes phases décrites ci-après.

### Phase 1 :

On prépare un solvant binaire comportant à titre de solvant principal un solvant alcoolique constitué d'un alcool inférieur tel que l'éthanol.

Et dans cette base, on incorpore et on met en solution un adjuvant constitué de polyéthylène glycol (PEG 6 000) introduit dans une proportion comprise entre 0,5 et 4 parts.

Dans le mélange ainsi constitué de 10 à 20 parts d'alcool éthylique (éthanol à 95 °) et de 0,5 à 2 parts de PEG, on incorpore 3 à 5 parts d'aspirine.

La proportion optimale se situant entre 6 et 8 parts du solvant binaire au minimum pour 1 part d'aspirine.

### Phase 2 :

On répartit cette solution d'aspirine de préférence sur un support de type sorbitol ou tout autre substrat soluble dans l'eau et utilisable dans la formulation de comprimés à sucer par l'intermédiaire des mélangeurs classiquement utilisés en pharmacie.
Le sorbitol ainsi mis en oeuvre possède une structure réticulée comme visible sur la photo numéro 1 annexée.

Dans ces conditions les particules du substrat formé de sorbitol sont imprégnées de la solution de manière totalement homogène tant au sein d'une même particule qu'au niveau des particules les unes par rapport aux autres.

Les particules de sorbitol imprégnées sont alors séchées dans un évaporateur sous vide à température ambiante.

Et le cycle d'imprégnation et d'évaporation est poursuivi à plusieurs reprises, jusqu'à ce que l'on atteigne la saturation optimale des microcristaux d'aspirine au sein du substrat de sorbitol.

### Phase 3 :

L'aspirine ainsi reconstituée sous-forme microdispersée au sein du substrat de sorbitol est, après tamisage, mélangée avec tout produit médicamenteux ou non destiné à améliorer l'efficacité thérapeutique, le goût ou la présentation et aussi facilitant les procédés techniques de fabrication.

### Phase 4 :

Après homogénéisation, le mélange constitué de particules ou granules de sorbitol contenant intérieurement les microcristaux d'aspirine, adhérant eux mêmes aux parois du sorbitol grâce à la présence du PEG servant de liant, est alors soumis à compression par les appareils classiques pour obtenir les pastilles, comprimés, tablettes, granules ou dragées.

Tout au long des différentes phases de mise en oeuvre du procédé, la présence du PEG permet de résoudre les problèmes de fabrication. Initialement, le PEG ne modifie pas la solubilité du principe actif (Aspirine) mais participe à limiter la reconstitution cristalline de l'aspirine au sein du substrat, et permet donc le meilleur état microcristallisé. Il permet aussi de donner au substrat une morphologie de granule spongiforme régulier, comme apparaît sur la photo numéro 2.
On évite aussi tout problème lié au démélange (manque d'homogénéité) des microcristaux d'aspirine par rapport au substrat ; le PEG résiduel et laissé sur place après évaporation du solvant principal constitue alors le liant qui permet l'adhésion ferme des microcristaux sur leur paroi d'adsorption.

On évite par conséquent dès le stade de l'évaporation, les pertes au niveau de la fabrication.

Les microcristaux étant régulièrement répartis au sein du substrat de sorbitol, cette répartition homogène subsiste et se retrouve par conséquent au niveau des produits finis dont la composition est ainsi maintenue constante.

Le PEG introduit par ailleurs un véritable enrobage des particules de sorbitol et cet enrobage permet en outre une très bonne plasticité du mélange lors de la phase de compression.

Tout au long de la vie du produit, l'homogénéité de l'ensemble étant assurée, on est certain de trouver au niveau de l'administration la qualité et la quantité de produit actif qui a été programmée.

Enfin, en assurant les cycles successifs d'enrichissement du substrat (chaque cycle comportant une phase d'imprégnation et une phase d'évaporation), on peut ajuster exactement le dosage des comprimés depuis 10 milligrammes d'acide acétylsalicylique par comprimé d'un poids de 300 à 500 mg.

Et ce dosage est adéquat pour obtenir un effet thérapeutique par voie locale notamment au contact des lésions ou par voie sublinguale au niveau d'affections générales organiques, comme, à titre d'exemple, certaines maladies cardiovasculaires.

Ainsi pour d'autres molécules, la quantité de principe actif pour un usage local ou général peut aussi être parfaitement ajustée.

Selon une autre caractéristique, on procède avant la phase finale de mise en comprimé et dans la formulation de ce dernier à l'incorporation d'un agent acidifiant tel que l'acide citrique.

Cet agent, lors de l'administration, permet d'acidifier légèrement le milieu de dissolution salivaire en l'établissant entre pH 3 et 4,5 ; ce milieu acide protège l'aspirine d'une attaque hydrolysante, augmente sa vitesse de dissolution dans la salive et facilite son contact et son passage à travers les muqueuses.

Selon un autre avantage de l'invention, le PEG a aussi réalisé un enrobage des microcristaux qui tout en les solidarisant fortement au substrat de sorbitol, les protège des phénomènes d'hydrolyse et de désacétylation à savoir la transformation de l'Acide Acétylsalicylique (aspirine) en acide Salicylique ceci avec production d'acide Acétique. Le PEG assure donc, non seulement une fixation physique des microcristaux, mais encore la protection de leur qualité chimique.

Selon un autre développement de l'invention, on introduit dans la formulation du comprimé, par incorporation au sorbitol chargé en microcristaux d'aspirine, des polymères hydrocolloïdes dans une proportion comprise entre 0,5 et 5 %.

La présence de ces polymères, après obtention de l'ensemble de microcristaux sur supports, permet de mettre en oeuvre une substance de type polymérique dont le rôle est de former une matrice hydrophile ou réseau gélifié ; ce dernier est ainsi hydraté en continu par la salive à la périphérie du comprimé et il se reconstitue aussitôt qu'il se défait au contact de la salive dans la cavité buccale.

On évite ainsi une libération trop rapide des cristaux d'aspirine qui entreraient directement au contact de la muqueuse et y exerceraient leur pouvoir agressif en raison de leur trop lente dissolution.

On permet ainsi une tolérance locale du produit considérablement améliorée puisque l'aspirine est ainsi partiellement et préalablement dissoute avant d'être libérée de la forme pharmaceutique ; permettant ainsi son assimilation rapide et évitant sa stagnation agressive au niveau du tissus muqueux.

Parmi les polymères hydrocolloïdes utilisables on citera, gomme arabique, gomme adragante, pectines (acide polygalacturonique), acides alginiques et dérivés, les carraghénanes, l'agar-agar, gomme guar et caroube.

Les dérivés de cellulose tels que, méthylcellulose, hydroxyéthycellulose, hydroxypropylcellulose , carboxyméthylcellulose, hydroxypropylméthylcellulose, méthyléthylcellulose.

Les polymères synthétiques tels que polyvinylpyrrolidone, polymères de l'acide carbovinylique (carbopol).

Des dérivés minéraux tels que bentonite, montmorillonite, weegum (marque déposée).

Les polymères d'origine biologique tels que : xanthane, gélatine, scléroglucane, dextran.

Les amidons modifiés tels carboxyméthylamidon, hydroxyéthylamidon, hydroxypropylamidon et leurs sels.

On décrit ci-après un procédé de fabrication :

### 1 - Formule unitaire

| COMPOSANT | QUANTITE | FONCTION |
|---|---|---|
| Acide acétylsalicylique | 100 mg | Principe actif |
| PEG 6000 | 125 mg | Liant-protecteur-régulateur de volume |
| Acide citrique | 10 mg | Correcteur de pH |
| Sorbitol type W60 | 950 mg | Support |
| Xanthane | 15 mg | Gélifiant |
| Aspartam | 7 mg | Edulcorant |
| Arôme orange | 10 mg | Arômatisant |
| Stéarylfumarate de Na | 20 mg | Lubrifiant |
| Ethanol à 96 % | V/V | Auxiliaire de fabrication. |

### 2 - Formule de fabrication

Pour un lot de 4 000 comprimés multiples ou sous-multiples :

| COMPOSANT | QUANTITES | FOURNISSEURS |
|---|---|---|
| Acide acétylsalicylique | 400 g | Rhône-Poulenc |
| PEG 6000 | 500 g | - |
| Acide citrique anhydre pulvérulent | 40 g | - |
| Sorbitol W 60 | 3 800 g | Roquette |
| Xanthane | 60 g | Kelco/Sanofi - Bio industrie |
| Aspartam | 28 g | Searle-SPCI |
| Arôme orange | 40 g | Robertet |
| Stéarylfumarate de Na | 116 g | - |
| Ethanol à 96 % V/V | 1 600 g | - |

### 3 - Matériel

1- Balance de portée 4 600 g et de précision 0.05 g, ce type METTLER PM.
2 - Balance de portée 36 kg et de précision 0,1 g, type METTLER PK 36.
3 - Cuve en acier inoxydable de 5 litres de capacité, munie d'une double enveloppe et d'un système de chauffage-refroidissement.
4 - Agitateur pneumatique type REXXON muni d'une pale d'agitation.
5 - Mélangeur granulateur-sécheur, type TOPO GRANULATEUR ou TURBOSPHERE MORITZ.
6 - Calibreur FREWITT muni d'une grille de 1,2 mm d'ouverture de maille.
7 - Mélangeur rotatif type roue RÖEHN équipé d'un container de 10 litres de capacité.
8 - Presse à comprimer type FETTE P 1000, munie de poinçons plats chanfreinés et de diamètre 19 mm.

### 4 - Mode opératoire

### Etape 1 :

Dans la cuve en acier inoxydable de 5 litres, introduire :
- Ethanol 96 % V/V 1 600 g.
Porter par circulation d'eau chaude dans la double enveloppe la température de l'alcool à 30°C +/- 2°C.

### Etape 2 :

Introduire dans l'Ethanol :
- PEG 6 000 500 g.
Agiter la solution pendant 15 minutes jusqu'à dissolution complète du PEG 6 000.
Lorsque le PEG 6 000 est entièrement dissout, refroidir la solution à + 18°C +/- 2°C.

### Etape 3 :

Sous agitation, introduire dans la solution précédente :
- Acide acétylsalicylique 400 g.

### Etape 4 :

Dans la cuve du mélangeur-granulateur introduire :
- Sorbitol W 60 préalablement démotté 3 800 g.
Après fermeture de l'appareil, homogénéiser le mélange à vitesse III pendant 5 minutes.
Porter la température de la double enveloppe de l'appareil à 50 °C Maintenir la vitesse de rotation sur III durant toute la fabrication.

### Etape 5 :

Lorsque la température du sorbitol atteint 30 °C, réduire la pression de la cuve du granulateur à moins 700 mbar.
Introduire par fraction de 100 ml la solution alcoolique d'acide acétylsalicylique.
Entre chaque fraction introduite, opérer le cycle de préséchage de 2 minutes selon les paramètres suivants :
- cassage du vide pendant 10 secondes et retour à la pression ambiante,
- rétablissement du vide à moins 700 mbar pendant 30 secondes.
Balancement du granulateur avec inversion du sens de rotation du mélangeur.

Durant toute l'opération d'imprégnation séchage, la température du mélange de sorbitol doit être comprise entre 28 et 30 degrés centigrades. (Tolérance 25° à 35°).

### Etape 6 :

Lorsque l'intégralité de la solution d'aspirine a été imprégnée sur le sorbitol, sécher la préparation durant 25 minutes selon le cycle utilisé à l'étape 5.

A la fin du séchage, la température du produit doit être comprise entre 35°C et 40°C.

### Etape 7 :

Ralentir la vitesse d'agitation à I et refroidir la préparation à température ambiante.

### Etape 8 :

Calibrer le mélange sec précédent sur un calibreur FREWITT muni d'une grille de 1,2 mm d'ouverture de maille.

Collecter le grain calibré dans la cuve du mélangeur roue RÖEHN.

### Etape 9 :

Introduire dans la cuve du mélangeur roue RÖEHN :
- aspartam
- xanthane
- arôme orange
- stéarylfumarate de Na.

Homogénéiser le mélange par 15 minutes d'agitation à la vitesse de 20 rotations par minute.

### Etape 10 :

Dans une pièce à hygrométrie contrôlée dont l'humidité relative est voisine de 20 %, comprimer le mélange préparé à l'étape précédente sur la presse rotative équipée de poinçons de diamètre 19 mm.

Régler les paramètres de la compression pour obtenir des comprimés de caractéristiques suivantes :
- poids moyen : 1 236 mg Tolérance : +/- 5%
- Dureté : 8 kg Tolérance : 6 à 12 kg

Après fabrication, les comprimés sont conditionnés selon les techniques habituelles propres à ce type de produit.
L'invention présente un domaine d'application très large. En effet, un nombre élevé de substances pharmaceutiques possède des caractéristiques physiques et/ou chimiques qui ne les prédisposent pas à une bonne dissolution dans les milieux aqueux de l'organisme.

En outre, certaines de ces substances sont de nature à irriter les tissus du tube digestif, elles y supportent mal la présence de substances alimentaires qui modifient leur biodisponibilité et, pour compenser ces défauts, l'obtention de l'effet thérapeutique peut nécessiter une posologie élevée.

Souvent, ces augmentations de doses génèrent elles-même des troubles accrus en fonction de la sensibilité personnelle du patient, sur le plan digestif ou général.

Il est donc intéressant pour des substances majeures comme les anti-inflammatoires, qui doivent traiter les affections stomatologiques et/ou otorhinolaryngologiques, de les mettre directement et localement en contact avec les lésions, tout en réduisant leur désagrément et leur agressivité pour les tissus.

Il est aussi intéressant, que ce soit pour des anti-inflammatoires, comme pour d'autres produits à vocation cardio-vasculaire, ou diurétique, ou pulmonaire, ou du système nerveux central, ou anti-virale, ou immunostimulante ou anticancéreux, ou à action hormonale ou ayant d'autres effets thérapeutiques, de pouvoir les administrer sans désagrément par la voie sublinguale.

Pour ce faire, le caractère agressif pour les tissus sensibles de la bouche ou désagréable pour le goût, desdites substances doit avoir été réduit mais aussi la meilleure dissolution possible dans la salive doit avoir été préparée, afin que la plus grande quantité de substance administrée passe par cette voie dans le délai le plus court.

Ceci exige que les principes qui président à la fabrication desdits composés permettent d'une part, cette meilleure et rapide dissolution que l'on attend, sans désagrément, et d'autre part, que ces principes de fabrication soient compatibles avec les exigences et moyens techniques habituels de l'industrie pharmaceutique, les contraintes de stabilité appliquées aux produits par la règlementation enfin et surtout compatibles avec les prix de revient industriels les plus bas possibles, car l'on connaît bien les exigences de faibles coûts rapportés aux médicaments.

Nous présentons ainsi un procédé qui dans sa totalité, résoud d'abord les problèmes de mise en oeuvre de principes actifs microadsorbés pour en permettre la meilleure dissolution dans la salive et réduire les désagréments desdites substances, le même procédé résoud aussi les problèmes techniques de fabrication rencontrés avec les substances microadsorbées et non encore résolus à ce jour et enfin ledit procédé ne surenchérit pas le prix de revient de ces produits innovants ainsi constitués qui apportent au Corps Médical et au public des facilités et simplicités nouvelles de traitements pour de nombreuses affections.

L'invention a été décrite plus particulièrement en rapport avec une présentation galénique d'aspirine.
En effet, cette substance cristalline, mal soluble dans l'eau, précipite et se cristallise activement dès qu'elle entre en contact avec le pH très acide (pH 1,5) de la muqueuse gastrique.

Cette recristallisation rapide est à l'origine des intolérances retrouvées chez la plupart des sujets, et il est bien démontré que les muqueuses gastriques en contact avec l'Aspirine saignent toutes, même les muqueuses saines.

Ainsi, si l'on doit traiter une inflammation bucco-dentaire ou pharyngo-laryngée, il paraît disproportionné de prendre de l'Aspirine par voie générale (réaction au niveau de l'estomac) alors qu'une quantité dix fois moindre d'Aspirine mise en contact avec la lésion suffit à produire localement l'effet anti-inflammatoire/antalgique recherché.

Ceci n'est cependant pas appliqué, car l'Aspirine est d'un goût désagréable et son acidité difficilement supportable.

De la même manière, il est bien connu que de petites quantités d'Aspirine (de 50 à 200 mg par jour) peuvent protéger efficacement de la survenue des thromboses des artères coronaires par le biais d'une action anti-agrégante.

La prise régulière et à long terme d'Aspirine pour ce but de prévention, se révèle là encore impossible à cause des effets irritants et saignements provoqués au niveau de l'estomac.

Le procédé qui est présenté permet de réaliser d'une part, des comprimés d'Aspirine à sucer traitant localement, efficacement et agréablement les inflammations bucco-pharyngo-laryngées, d'autre part, de proposer la prise régulière de comprimés d'Aspirine issus du même procédé d'un dosage de 20 à 150 mg par voie sublinguale dans la prévention du risque cardio-vasculaire, ceci là encore sans aucun désagrément local ou de goût. D'autres substances complétant cette action spécifique peuvent aussi être adjointes à l'Aspirine.

D'autres substances encore peuvent utilement disposer du procédé pour améliorer leur biodisponibilité et leur efficacité thérapeutique ; à titre d'exemple, la nifédipine peu soluble dans l'eau, présentée sous forme de gélules à dissolution/libération entérale.

Or, cet anti-coronarien majeur peut protéger de la survenue d'un infarctus brutal dès les premiers signes, à condition d'être rapidement admis dans la circulation. Dans ce cas particulier, un comprimé issu du procédé peut être rapidement dissous sous la langue et la substance active, la nifédipine passe en quelques secondes dans le torrent circulatoire pour agir aussitôt au niveau du coeur.

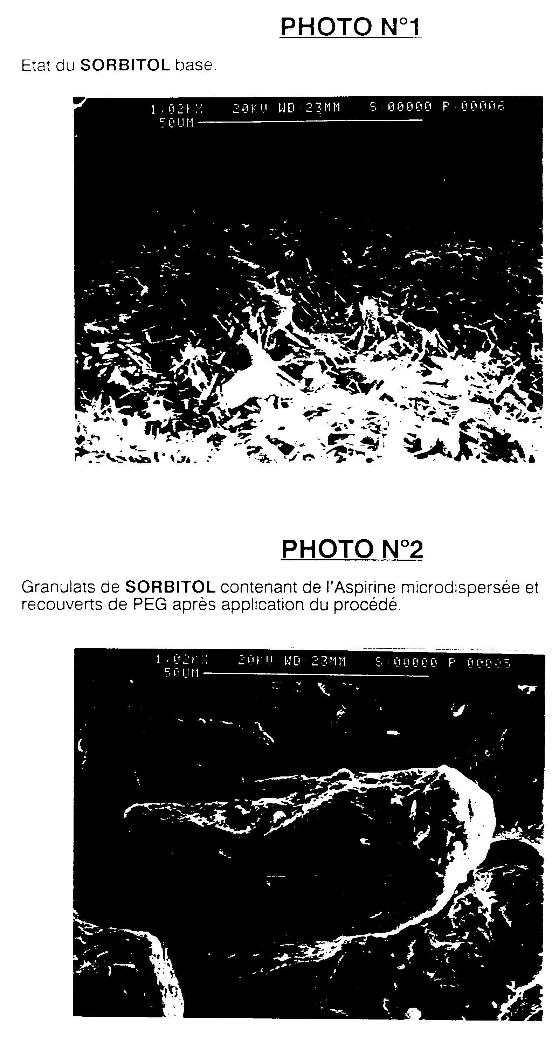

## Revendications

1. Procédé de préparation d'une composition thérapeutique à usage interne, notamment à administration per os, sous présentation galénique, et contenant au moins un principe actif sous forme solide adsorbé sur un substrat, le produit actif y étant réparti à l'état de microparticules finement dispersées et le procédé étant de type comportant les phases :
- de mise en solution du principe actif de départ dans un solvant approprié,
- d'imprégnation de la solution ainsi formée sur un support alimentaire de structure appropriée et pharmaceutiquement compatible avec ledit principe actif,
- d'évaporation du solvant de la solution adsorbée au sein dudit support en provoquant la reconstitution du principe actif par la formation de microcristaux adsorbés sur les parois et au sein du substrat,
- caractérisé en ce que le solvant est constitué d'un système binaire comportant un solvant principal, de nature organique, et un adjuvant, de type solide à la température ambiante et soluble dans le solvant principal, l'adjuvant présentant par ailleurs des propriétés lubrifiantes, la présence de l'adjuvant solide au sein du substrat, après évaporation du solvant principal assurant une limitation de l'envergure cristalline des microcristaux et leur cimentation sur les parois d'adsorption évitant leur entraînement parasite.

2. Procédé selon la revendication 1, caractérisé en ce que le substrat possède au départ une structure réticulée devenant spongieuse et alvéolaire, après mise en oeuvre du procédé, par imprégnation de l'adjuvant déposé dans la structure réticulée du substrat après évaporation du solvant principal enserrant les microcristaux reconstitués du principe actif.

3. Procédé selon la revendication 1 ou la revendication 2, caractérisé en ce que le substrat, après absorption de la solution, est soumis à une opération d'évacuation du solvant à température ambiante par réduction de la pression atmosphérique, c'est-à-dire par séchage sous vide de sorte que le substrat acquiert par cette mise en oeuvre une structure poreuse spongieuse et régulière qui recèle les microcristaux de principe actif.

4. Procédé selon l'une des revendications 1 ou 2, caractérisé en ce que la phase de conditionnement comporte l'homogénéisation du substrat suivie d'une compression en vue de la formation de doses pour prises unitaires et constituant des pastilles, comprimés, tablettes, granulés ou dragées.

5. Application du procédé selon l'une des revendications 1 à 3 applicable à la préparation d' une spécialité contenant une substance thérapeutique faiblement hydrosoluble notamment antalgique ou anti-inflammatoire et notamment à usage local, à base d'acide acétylsalicylique (aspirine) ou à assimilation directe par les voies des muqueuses sublinguales.

6. Procédé selon l'une des revendications 1 à 3, caractérisé en ce que le solvant binaire utilisé comporte à titre de solvant principal un alcool à faible tension de vapeur, et plus spécialement un alcool monofonctionnel inférieur choisi dans la famille comportant le méthanol, l'éthanol, le butanol et le propanol.

7. Procédé selon la revendication 6, caractérisé en ce que l'on utilise un alcool possédant au départ une concentration de préférence supérieure à 90°.

8. Procédé selon l'une des revendications 1 à 3, caractérisé en ce que l'adjuvant intégré dans le solvant binaire et mis en solution dans ce dernier est constitué d'un polymère organique choisi pour ne pas limiter la solubilité du produit actif dans le solvant principal.

9. Procédé selon la revendication 8, caractérisé en ce que l'adjuvant est constitué d'un polymère organique, soluble à la fois dans les alcools inférieurs et dans l'eau.

10. Procédé selon l'une des revendications 8 ou 9 et caractérisé en ce que l'adjuvant est plus spécialement choisi dans la famille des polymères comportant :
- les dérivés des Glycérides tels que mono et diglycérides.
- les Polyéthylènes Glycols (PEG) de différents poids moléculaires de préférence compris entre 1500 et 8000.
- Esters et Ethers des alcoolamines
- Esters et Ethers de Diethanolamines
- Esters et Ethers des Monoisopropanolamines
- Esters et Ethers des Isopropanolamines
- Esters et Ethers des Monoéthanolamines
Ethoxylamine
Dérivés éthoxylés des acides gras
Polyéther-alcool d'alkylamyl
Polyéther-alcool d'alkanolamide
Esters acides de mono et diglycérides
Esters d'acides gras
Esters du Polyéthylène Glycol (PEG), notamment monolaurate, stéarate, distéarate de PEG.
Esters et Ethers du Propylène Glycol
Esters et Ethers du Polyglycérol
Esters et Ethers du Polyglycol
Esters et Ethers du Glycol
Dérivés Ethoxylés des alcools et alcools gras et Phénols
Dérivés Propoxylés des alcools gras alkylphénols.
Ethoxyalkylphénol.
Esters du Sorbitane notamment Monostéarate de Sorbitane.
Esters du Saccharose notamment Palmitate et Stéarate de Saccharose.
Polyoxyéthers de Glycol, de Sorbitane, de Glycérol.
Lécithines et dérivés.
Alkyloamide.
Huile de Castor éthoxylée hydrogénée.
Esters du Sorbitane éthoxylé.
Esters de l'éthylène glycol.
Copolymères et polymères de l'oxyde d'Ethylène et de Propylène.
Dérivés des éthanolamines d'acides gras.
Dérivés des diéthanolamines d'acides gras.
Polycondensats d'éthylène oxyde.
Polycondensats de polypropylène glycol.

11. Procédé selon l'une des revendications 1 à 10, caractérisé en ce que l'adjuvant est introduit dans une proportion comprise entre 10 à 60% du poids total du solvant binaire.

12. Procédé selon l'une des revendications 1 à 11, caractérisé en ce que l'adjuvant est introduit dans le système solvant binaire dans une proportion comprise entre 10 et 200mg pour un comprimés de 1,4g.

13. Procédé selon l'une des revendications 1 à 12 caractérisé en ce que le substrat est choisi dans la famille comportant les hydrates de carbone alimentaires.

14. Procédé selon la revendication 13, caractérisé en ce que le substrat est constitué par du Sorbitol ou du Mannitol.

15. Procédé selon l'une des revendications 1 à 14, caractérisé en ce que l'imprégnation et l'absorption de la solution par le substrat est poursuivie pour tendre à une saturation et obtention d'une répartition homogène de la solution absorbée dans le substrat.

16. Procédé selon l'une des revendications 1 à 15, caractérisé en ce que le substrat subit plusieurs cycles successifs d'enrichissement en microcristaux, chaque cycle comportant une phase d'imprégnation suivie d'une phase d'évaporation, les cycles étant répétés jusqu'à ce que la saturation en microcristaux soit optimale.

17. Spécialité médicamenteuse sous présentation galénique et constituée notamment d'une pastille, granule, comprimé ou analogue, apte à être sucée par l'utilisateur ou à administration sublinguale, caractérisé en ce qu'il comporte au moins un produit actif sous forme microdispersée au sein du substrat, cette microdispersion ayant été obtenue conformément au procédé selon l'une des revendications 1 à 16.

18. Spécialité médicamenteuse selon la revendication 17, caractérisée en ce qu'elle comporte un produit actif microdispersé au sein d'un substrat et inclus dans la structure spongieuse ou alvéolaire de ce dernier sous forme de microcristaux, la taille des microcristaux étant généralement inférieure à 10 microns.

19. Spécialité médicamenteuse selon la revendication 17 ou la revendication 18, caractérisée en ce que le produit actif est constitué par une substance thérapeutique faiblement hydrosoluble, notamment de l'acide acétylsalicylique (aspirine) reconstituée sous forme de microcristaux au sein d'un support constitué de sorbitol ou de mannitol.

20. Spécialité médicamenteuse selon la revendication 19, caractérisée en ce qu'elle comporte un agent acidifiant tel que l'acide critique, ou acidifiant équivalent.

21. Spécialité médicamenteuse selon la revendication 19, caractérisée en ce que la composition de chaque tablette ou comprimé est basée sur un pourcentage d'acide acétylsalicylique (aspirine) sous forme de microcristaux compris entre 10 et 200 milligrammes sur un substrat de Sorbitol de poids compris entre 100 et 2500 milligrammes.

22. Spécialité médicamenteuse selon l'une des revendications 18 ou 19 ou 20, caractérisée en ce qu'elle comporte seule ou en combinaison synergique outre de l'acide acétylsalicylique (aspirine) sous forme microdispersée, un ou plusieurs produits actifs auxiliaires choisis dans les familles comportant :
- des antiseptiques (tels les ammoniums quaternaires, la chlorexhidrine)
- des antibiotiques (tels que bacitracine, tyrothricine, fusafungine)
- des antifongiques locaux (tels que miconazole, nystatine, amphotéricine B)
- des antiviraux (tels l'Acyclorir, l'AZT, l'interféron alpha)
- des antiagrégants (ticlopidine, prostacycline, flurbiprofène, dipyramidole)
- des cicatrisants (allantoïne, azulène)
- des enzymes (els : lysozyme, papaïne, bromeline)
- des analgésiques (tels que la procaïne, la téracaïne, la stovaïne).

23. Spécialité médicamenteuse selon l'une des revendications 18,19,20 ou 21, caractérisée en ce qu'elle comporte par incorporation au sorbitol ou au mannitol chargé en microcristaux d'acide acétylsalicylique (aspirine), des polymères hydrocolloïdes dans une proportion comprise entre 0,5 et 5%.

24. Spécialité médicamenteuse selon la revendication 23, caractérisée en ce que les polymères hydrocolloïdes sont choisis dans le groupe comportant les produits suivants : gomme arabique, gomme adragante, pectines (acide polygalacturonique), des acides alginiques et dérivés, les carraghénanes, l'agar-agar, gomme de guar et caroube;
Les dérivés de la cellulose tels que méthylcellulose, hydroxyéthylcellulose,hydroxypropylcellulose, carboxyméthylcellulose, hydroxypropylméthylcellulose, méthyléthylcellulose;
Les polymères synthétiques tels que polyvinylpyrrolidone, polymère de l'acide carbovinylique (carbopol);
Des dérivés minéraux tels que bentonite, montmorillonite, weegum (marque déposée);
Les polymères d'origine biologique tels que :
xanthane, gélatine, scléroglucane, dextran;
Les amidons modifiés tels carboxyméthylamidon, hydroxyéthylamidon, hydroxypropylamidon et leurs sels.

25. Spécialité médicamenteuse selon l'une des revendications 17 à 24, caractérisée en ce qu'elle comporte un pourcentage de principe actif sous forme de cristaux compris entre 2 et 200 mg, pouvant être constitué par des substances thérapeutiques appartenant aux familles du bépridil, de la molsidomine, de l'azidotymidine, de l'interféron alpha, de l'acyclovir ou autres substances anti-virales ou toutes autres substances médicamenteuses pouvant être administrées par voie sublinguale pour une thérapeutique générale, comme les substances actives sur le système nerveux central, comme encore les substances peptidiques à action anti-cancéreuse ou hormonale et toutes autres substances fragiles possédant une demi-vie courte et nécessitant des prises répétées pour maintenir leur activité organique constante.

26. Spécialité médicamenteuse selon les revendications 17 à 25, caractérisée en ce qu'elle comporte une association de différents principes actifs microcristallisés au sein du même substrat ou associés en cours de fabrication, soit en présence, soit en l'absence d'acide acétylsalicylique (aspirine).

## Claims

1. Method for preparing a therapeutical composition for internal use, particularly by administration per os, in galenic form, and containing at least one active principle in solid form adsorbed on a substrate, the active product being distributed thereon in the state of finely dispersed microparticles and the method being of type comprising the phases:
- of dissolving the starting active principle in an appropriate solvent,
- of impregnating the solution thus formed on an alimentary support of appropriate structure and pharmaceutically compatible with said active principle,
- of evaporating the solvent from the solution adsorbed within said support, provoking the reconstitution of the active principle by the formation of microcrystals adsorbed on the walls and within the substrate, characterized in that the solvent is constituted by a binary system comprising a principal solvent, of organic nature, and an additive, of solid type at ambient temperature and soluble in the principal solvent, the additive furthermore presenting lubricating properties, the presence of the solid additive within the substrate, after evaporation of the principal solvent ensuring a limitation of the crystalline spread of the microcrystals and their cementation on the adsorption walls avoiding their parasitic entrainment.

2. Method according to Claim 1, characterized in that the substrate presents at the start a cross-linked structure becoming spongy and alveolar, after implementation of the method, by impregnation of the additive deposited in the cross-linked structure of the substrate after evaporation of the principal solvent embracing the reconstituted microcrystals of the active principle.

3. Method according to Claim 1 or Claim 2, characterized in that the substrate, after absorption of the solution, is subjected to an operation of evacuation of the solvent at ambient temperature by reduction of the atmospheric pressure, i.e. by drying in vacuo so that the substrate acquires by such implementation a porous, spongy and regular structure which contains the microcrystals of active principle.

4. Method according to one of Claims 1 or 2, characterized in that the phase of packaging comprises the homogenization of the substrate followed by a compression with a view to the formation of unitary doses and constituting pastilles, tablets, lozenges, granules or pills.

5. Application of the method according to one of Claims 1 to 3 applicable to the preparation of a specialty containing a weakly water-soluble therapeutical substance, particularly antalgic or anti-inflammatory and particularly for local use, based on acetylsalicylic acid (aspirin) and with direct assimilation by the sublingual mucous route.

6. Method according to one of Claims 1 to 3, characterized in that the binary solvent used comprises by way of principal solvent an alcohol with low vapour tension, and more especially a monofunctional lower alcohol chosen from the family comprising methanol, ethanol, butanol and propanol.

7. Method according to Claim 6, characterized in that an alcohol is used, presenting at the start a concentration preferably greater than 90°.

8. Method according to one of Claims 1 to 3, characterized in that the additive integrated in the binary solvent and dissolved therein is constituted by an organic polymer chosen in order not to limit the solubility of the active product in the principal solvent.

9. Method according to Claim 8, characterized in that the additive is constituted by an organic polymer soluble both in lower alcohols and in water.

10. Method according to one of Claims 8 or 9 and characterized in that the additive is more especially chosen from the family of polymers comprising:
- derivatives of Glycerides such as mono and diglycerides,
- Polyethylene Glycols (PEG) of different modular weights preferably included between 1500 and 8000,
- Esters and Ethers of alcohol-amines
- Esters and Ethers of Diethanolamines
- Esters and Ethers of Monoisopropanolamines
- Esters and Ethers of Isopropanolamines
- Esters and Ethers of Monoethanolamines
Ethoxyamine
Ethoxylated derivatives of fatty acids
Polyether-alcohol of alkylamyl
Polyether-alcohol of alkanolamide
Acid esters of mono and diglycerides
Esters of fatty acids
Esters of Polyethylene Glycol (PEG), particularly monolaurate, stearate, distearate of PEG
Esters and Ethers of Propylene Glycol
Esters and Ethers of Polyglycerol
Esters and Ethers of Polyglycol
Esters and Ethers of Glycol
Ethoxylated derivatives of alcohols and fatty alcohols and Phenols
Propoxylated derivatives of fatty alcohols alkylphenols Ethoxyalkylphenol
Esters of Sorbitane, particularly Monostearate of Sorbitane
Esters of Saccharose particularly Palmitate and Steerate of Saccharose
Polyoxyethers of Glycol, of Sorbitane, of Glycerol
Lecithins and derivatives
Alkyloamide
Ethoxylated, hydrogenated castor oil
Esters of ethoxylated Sorbitane
Esters of ethylene glycol
Copolymers and polymers of Ethylene and Propylene oxide
Derivatives of ethanolamines of fatty acids
Derivatives of diethanolamines of fatty acids
Polycondensates of ethylene oxide
Polycondensates of polypropylene glycol.

11. Method according to one of Claims 1 to 10, characterized in that the additive is introduced in a proportion included between 10 to 60% of the total weight of the binary solvent.

12. Method according to one of Claims 1 to 11, characterized in that the additive is introduced in the binary solvent system in a proportion included between 10 and 200 mg for a tablet of 1.4 g.

13. Method according to one of Claims 1 to 12, characterized in that the substrate is chosen from the family comprising alimentary carbohydrates.

14. Method according to Claim 13, characterized in that the substrate is constituted by Sorbitol or Mannitol.

15. Method according to one of Claims 1 to 14, characterized in that the impregnation and absorption of the solution by the substrate is continued to tend to a saturation and obtaining of a homogeneous distribution of the solution absorbed in the substrate.

16. Method according to one of Claims 1 to 15, characterized in that the substrate undergoes a plurality of successive cycles of enrichment in microcrystals, each cycle comprising a phase of impregnation followed by a phase of evaporation, the cycles being repeated until saturation in microcrystals is optimum.

17. Galenically presented medicamental specialty constituted in particular by a pastille, granule, tablet or the like, adapted to be sucked by the user or for sublingual administration, characterized in that it comprises at least one active product in form microdispersed within the substrate, such microdispersion having been obtained in accordance with the method according to one of Claims 1 to 16.

18. Medicamental specialty according to Claim 17, characterized in that it comprises an active product microdispersed within a substrate and included in the spongy or alveolar structure thereof in the form of microcrystals, the size of the microcrystals generally being less than 10 microns.

19. Medicamental specialty according to Claim 17 or Claim 18, characterized in that the active product is constituted by a weakly water-soluble therapeutical substances, particularly acetylsalicylic acid (aspirin) reconstituted in the form of microcrystals within a support constituted by sorbitol or mannitol.

20. Medicamental specialty according to Claim 19, characterized in that it comprises an acidifying agent such as critical (sic) acid, or equivalent acidifiant.

21. Medicamental specialty according to Claim 19, characterized in that the composition of each tablet or lozenge is based on a percentage of acetylsalicylic acid (aspirin) in the form of microcrystals included between 10 and 200 milligrams on a substrate of Sorbitol of weight included between 100 and 2500 milligrams.

22. Medicamental specialty according to one of Claims 18 or 19 or 20, characterized in that it comprises, alone or in synergic combination, in addition to the acetylsalicylic acid (aspirin) in microdispersed form, one or more auxiliary active products chosen from the families comprising:
- antiseptics (such as quaternary ammoniums, chorexhidrine)
- antibiotics (such as bacitracine, tyrothricine, fusafungine)
- local antifungics (such as micronazol, nystatine, amphotericine B)
- antivirals (such as Acyclorir, AZT, alpha interferon)
- anti-aggregants (ticlopidine, prostacycline, flurbiprofene, dipyramidole)
- cicatrizants (allantoin, azulene)
- enzymes (such as lysozyme, papain, bromeline)
- analgesics (such as procaine, teracaine, stovaine).

23. Medicamental specialty according to one of Claims 18, 19, 20 or 21, characterized in that it comprises by incorporation with sorbitol or mannitol charged with microcrystals of acetylsalicylic acid (aspirin), hydrocolloid polymers in a proportion included between 0.5 and 5%.

24. Medicamental specialty according to Claim 23, characterized in that the hydrocolloid polymers are chosen from the group comprising the following products: gum arabic, gum tragacanth, pectines (polygalacturonic acid), alginic acids and derivatives, carragheenins, agar-agar, gum of guar and carob;
The derivatives of cellulose such as methylcellulose, hydroxyethylcellulose, hydroxypropylcellulose, carboxymethylcellulose, hydroxypropylmethylcellulose, methylethylcellulose;
Synthetic polymers such as polyvinylpyrrolidone, polymer of carbovinylic acid (carbopol);
Inorganic derivatives such as bentonite, montmorillonite, weegum (Registered Trademark);
Polymers of biological origin such as xanthane, gelatin, scleroglucane, dextran);
Modified starches such as carboxymethyl starch, hydroxyethyl starch, hydroxypropyl starch and salts thereof.

25. Medicamental specialty according to one of Claims 17 to 24, characterized in that it comprises a percentage of active principle in the form of crystals included between 2 and 200 mg, being able to be constituted by therapeutical substances belonging to the families of bepridil, molsidomine, azidotymidine, alpha interferon, acyclovir or other anti-viral substances or any other medicamental substances which may be administered by the sublingual route for general therapeutics, such as substances active on the central nervous system, such as, further, peptidic substances with anti-cancerous or hormonal action and all other fragile substances presenting a short half-life and requiring repeated doses to maintain their organic activity constant.

26. Medicamental specialty according to Claims 17 to 25, characterized in that it comprises an association of different active principles microcrystallized within the same substrate or associated in the course of manufacture, either in the presence, or in the absence of acetylsalicylic acid (aspirin).

## Patentansprüche

1. Verfahren zur galenischen Herstellung eines therapeutischen Mittels zur inneren Anwendung, insbesondere für die perorale Verabreichung, in galenischer Form mit mindestens einem in fester Form vorliegenden, auf einem Substrat adsorbierten Wirkprinzip, wobei der Wirkstoff in Form von Mikropartikeln fein verteilt ist und das Verfahren die folgenden Phasen beinhaltet:
- Lösen des Ausgangs-Wirkprinzips in einem geeigneten Lösungsmittel;
- Aufbringen der so hergestellten Lösung auf einen pharmazeutisch mit dem Wirkprinzip verträglichen Nahrungsmittel-Träger geeigneter Struktur;
- Verdampfen des Lösungsmittels der vom genannten Träger adsorbierten Lösung unter Wiederherstellung des Wirkprinzips durch Ausbildung von an den Wandungen des Substrats und in diesem adsorbierten Mikrokristallen;
- dadurch gekennzeichnet, daß das Lösungsmittel aus einem binären System besteht mit einem Hauptlösungsmittel organischer Art und einem Zusatz-Lösungsmittel, das bei Umgebungstemperatur und im Hauptlösungsmittel lösbar ist, wobei das Zusatz-Lösungsmittel im übrigen Schmiereigenschaften aufweist und die Anwesenheit des festen Zusatz-Lösungsmittels im Substrat nach dem Verdampfen des Hauptlösungsmittels eine Begrenzung der kristallinen Größe der Mikrokristalle sicherstellt und durch deren feste Verbindung mit den Adsorptionswandungen verhindert, daß sie in unerwünschter Weise mitgeschleppt werden.

2. Verfahren nach Anspruch 1, dadurch gekennzeichnet, daß das Substrat zunächst eine netzartige Struktur aufweist, die nach der Durchführung des Verfahrens, nach dem Eintrag des in der netzartigen Struktur des Substrats nach dem Verdampfen des Hauptlösungsmittels eingelagerten Zusatz-Lösungsmittels, die wiederhergestellten Mikrokristalle des Wirkprinzips in sich einschließt.

3. Verfahren nach Anspruch 1 oder Anspruch 2, dadurch gekennzeichnet, daß das Substrat nach Absorption der Lösung einer Absaugung des Lösungsmittels bei Umgebungstemperatur durch Verminderung des atmosphärischen Drucks, d.h. einem Vakuum-Trocknungsverfahren unterzogen wird, so daß das Substrat durch diesen Arbeitsgang eine poröse, schwammartige und regelmäßige Struktur annimmt, die die Mikrokristalle des Wirkprinzips in sich einschließt.

4. Verfahren nach einem der Ansprüche 1 oder 2, dadurch gekennzeichnet, daß die Konditionierphase die Homogenisierung des Substrats und einen anschließenden Preßvorgang zur Herstellung von Einnahme-Einheiten in Form von Pastillen, Pillen, Tabletten, Granulaten oder Dragees umfaßt.

5. Anwendung des Verfahrens nach einem der Ansprüche 1 bis 3 für die Herstellung einer Spezialität, die eine schwach wasserlösliche therapeutische Substanz, insbesondere eine schmerzstillende oder entzündungshemmende Substanz, besonders für die örtliche Anwendung auf der Grundlage von Acetylsäure (Aspirin) oder für die direkte Aufnahme über die sublingualen Schleimhäute enthält.

6. Verfahren nach einem der Ansprüche 1 bis 3, dadurch gekennzeichnet, daß das verwendete binäre Lösungsmittel als Hauptlösungsmittel einen Alkohol geringer Dampfspannung, insbesondere einen geringwertigen monofunktionellen Alkohol aus der Methanol, Ethanol, Butanol und Propanol enthaltenden Familie enthält.

7. Verfahren nach Anspruch 6, dadurch gekennzeichnet, daß ein Alkohol verwendet wird, der zunächst eine Konzentration von vorzugsweise über 90° aufweist.

8. Verfahren nach einem der Ansprüche 1 bis 3, dadurch gekennzeichnet, daß das im binären Lösungsmittel enthaltene und darin gelöste Zusatz-Lösungsmittel aus einem organischen Polymer besteht, das derart gewählt wird, daß die Löslichkeit des Wirkstoffs im Hauptlösungsmittel nicht eingeschränkt wird.

9. Verfahren nach Anspruch 8, dadurch gekennzeichnet, daß das Zusatz-Lösungsmittel aus einem organischen Polymer besteht, das gleichzeitig in geringwertigen Alkoholen und in Wasser löslich ist.

10. Verfahren nach einem der Ansprüche 8 oder 9, dadurch gekennzeichnet, daß das Zusatz-Lösungsmittel insbesondere aus der die folgenden Polymere enthaltenden Polymerfamilie gewählt wird:
- Glyceridderivate, wie Mono- und Diglyceride;
- Polyethylenglycole (PEG) unterschiedlicher Molekulargewichte, vorzugsweise mit einem Molekulargewicht zwischen 1500 und 8000;
- Alkoholaminester und -ether
- Diethanolaminester und -ether
- Monoisopropanolaminester und -ether
- Isopropanolaminester und -ether
- Monoethanolaminester und -ether
Ethoxylamin
Ethoxylierte Fettsäurederivate
Alkylamyl-Polyetheralkohol
Alkanolamid-Polyetheralkohol
Mono- und Diglyceridsäureester
Fettsäureester
Polyethylenglycolester (PEG), insbesondere PEG-Monolaurat, -Stearat, -Distearat.
Propylenglycolester und -ether
Polyglycerolester und -ether
Polyglycolester und -ether
Glycolester und -ether
Ethoxilierte Alkohol-, Fettalkohol- und Phenolderivate Propoxylierte Fettalkohol-Alkylphenolderivate.
Ethoxyalkylphenol.
Sorbitanester, insbesondere Sorbitanmonostearat.
Saccharoseester, insbesondere Saccharosepalmitat und -stearat.
Glycol-, Sorbitan- Glycerol-Polyoxyether.
Lezithine und deren Derivate.
Alkyloamid.
Ethoxyliertes, gehärtetes Castoröl.
Ethoxylsorbitanester.
Ethylenoxid- und Propylen-Copolymere und -Polymere.
Fettsäure-Ethanolaminderivate.
Fettsäure-Diethanolaminderivate.
Ethylenoxidpolykondensate.
Polypropylenglycolpolykondensate.

11. Verfahren nach einem der Ansprüche 8 bis 10, dadurch gekennzeichnet, daß das Zusatz-Lösungsmittel in einem Verhältnis zwischen 10 und 60 % des Gesamtgewichts des binären Lösungsmittels zugegeben wird.

12. Verfahren nach einem der Ansprüche 8 bis 11, dadurch gekennzeichnet, daß das Zusatz-Lösungsmittel dem binären Lösungsmittel in einem Anteil von zwischen 10 und 200 mg pro Tablette von 1,4 g zugegeben wird.

13. Verfahren nach einem der Ansprüche 1 bis 12, dadurch gekennzeichnet, daß das Substrat aus der Familie der Nahrungsmittel-Kohlehydrate gewählt wird.

14. Verfahren nach Anspruch 13, dadurch gekennzeichnet, daß das Substrat aus Sorbitol oder Mannitol besteht.

15. Verfahren nach einem der Ansprüche 1 bis 14, dadurch gekennzeichnet, daß das Tränken des Substrats mit der Lösung und deren Absorption durch das Substrat mit dem Ziel einer Sättigung und homogenen Verteilung der absorbierten Lösung im Substrat durchgeführt wird.

16. Verfahren nach einem der Ansprüche 1 bis 15, dadurch gekennzeichnet, daß das Substrat mehreren aufeinanderfolgenden Anreichungszyklen mit Mikrokristallen unterzogen wird, wobei jeder Zyklus eine Tränkphase mit nachfolgender Verdampfungsphase umfaßt und diese Zyklen solange wiederholt werden, bis eine optimale Sättigung mit Mikrokristallen erreicht ist.

17. Arzneimittelspezialität in galenischer Form, insbesondere bestehend aus einer Pastille, einem Granulat, einer Tablette oder dergleichen zum Lutschen oder zur sublingualen Verabreichung, dadurch gekennzeichnet, daß sie mindestens einen Wirkstoff in im Substrat mikrofein verteilter Form enthält, wobei diese mikrofeine Verteilung nach dem Verfahren nach einem der Ansprüche 1 bis 16 erzielt wird.

18. Arzneimittelspezialität nach Anspruch 17, dadurch gekennzeichnet, daß sie einen Wirkstoff enthält, der im Substrat mikrofein verteilt und in der schwammartigen oder zellartigen Struktur des Substrats in Form von Mikrokristallen eingeschlossen ist, deren Größe im allgemeinen unter 10 µm liegt.

19. Arzneimittelspezialität nach Anspruch 17 oder Anspruch 18, dadurch gekennzeichnet, daß der Wirkstoff aus einer schwach wasserlöslichen therapeutischen Substanz, insbesondere Acetylsäure (Aspirin) besteht, die in Form von Mikrokristallen innerhalb eines aus Sorbitol oder Mannitol bestehenden Substrats wiederhergestellt wurde.

20. Arzneimittelspezialität nach Anspruch 19, dadurch gekennzeichnet, daß sie ein Säuerungsmittel, wie Zitronensäure oder ein entsprechendes Säuerungsmittel, enthält.

21. Arzneimittelspezialität nach Anspruch 19, dadurch gekennzeichnet, daß die Grundlage für die Zusammensetzung jeder Tablette oder Pastille Acetylsäure (Aspirin) in Form von Mikrokristallen mit einem Anteil von zwischen 10 und 200 mg bei einem Sorbitol-Substratgewicht zwischen 100 und 2500 mg ist.

22. Arzneimittelspezialität nach einem der Ansprüche 18, 19 oder 20, dadurch gekennzeichnet, daß sie allein oder in synergetischer Kombination neben der Acetylsäure (Aspirin) in mikrofeiner Verteilung einen Hilfswirkstoff oder mehrere Hilfswirkstoffe aus folgenden Familien enthält:
- Antiseptika (wie quartäre Ammoniumverbindungen, Chlorexhydrin)
- Antibiotika (wie Bacitracin, Thyrothricin, Fusafungin)
- Lokale Antimykotika (wie Miconazol, Nystatin, Amphotericin B)
- Antivirale Mittel (wie Acyclorir, AZT, Interferon Alpha)
- Antiaggregierend wirkende Stoffe (Ticlopidin, Prostacyclin, Flurbiprofen, Dipyramidol)
- Narbenbildungsfördernde Mittel (Allantoin, Azulen)
- Enzyme (wie Lysozym, Papain, Bromelin)
- Analgetika (wie Procain, Teracain, Stovain).

23. Arzneimittelspezialität nach einem der Ansprüche 18, 19, 20 oder 21, dadurch gekennzeichnet, daß sie durch Einbau in Acetylsäure (Aspirin) in mikrokristalliner Form enthaltendes Sorbitol oder Mannitol hydrocolloide Polymere in einem Anteil von zwischen 0,5 und 5 % enthält.

24. Arzneimittelspezialität nach Anspruch 23, dadurch gekennzeichnet, daß die hydrocolloiden Polymere aus der Gruppe der folgenden Stoffe gewählt werden: Gummi arabicum, Tragantgummi, Pektine (Polygalacturonsäure), Alginsäuren und deren Derivate, Carragheenane, Agar-Agar, Guaran und Johannisbrotgummi;
Zellulosederivate, wie Methylzellulose, Hydroxyethylzellulose, Hydroxypropylzellulose, Carboxymethylzellulose, Hydroxypropylmethylzellulose, Methylethylzellulose;
Synthetische Polymere, wie Polyvinylpyrrolidon, Carbovinylsäurepolymer (Carbopol);
Mineralische Derivate, wie Bentonit, Montmorillonit, Weegum (eingetragenes Warenzeichen);
Polymere biologischen Ursprungs, wie Xanthan, Gelatine, Scleroglucan, Dextran;
Modifizierte Amidone, wie Carboxymethylamidon, Hydroxyethylamidon, Hydroxypropylamidon und deren Salze.

25. Arzneimittelspezialität nach einem der Ansprüche 17 bis 24, dadurch gekennzeichnet, daß sie einen Anteil des Wirkstoffs in kristalliner Form von zwischen 2 und 200 mg enthält, wobei der Wirkstoff aus therapeutischen Substanzen der Familien Bepridil, Molsidomin, Azydotymidin, Interferon Alpha, Acyclovir oder anderen antiviralen Substanzen oder aus allen anderen Arzneimittelsubstanzen bestehen kann, die sublingual im Rahmen einer allgemeinen Therapie verabreicht werden können, wie auf das zentrale Nervensystem wirkende Wirkstoffe oder auch peptische Substanzen mit krebshemmender oder hormonaler Wirkung und alle sonstigen empfindlichen Substanzen, die eine kurze Halbwertzeit besitzen und wiederholte Einnahmen erfordern, um eine konstante organische Wirkung zu erzielen.

26. Arzneimittelspezialität nach den Ansprüchen 17 bis 25, dadurch gekennzeichnet, daß sie gleichzeitig mehrere Wirkprinzipien enthält, die in mikrokristalliner Form in ein und demselben Substrat vorliegen oder während des Herstellungsverfahrens in Anwesenheit oder Abwesenheit von Acetylsäure (Aspirin) vereinigt werden.
